# EUROPEAN PATENT SPECIFICATION

(11) **EP 3 287 782 B1**
(45) Date of publication and mention of the grant of the patent: **22.04.2020**
(21) Application number: 17192746.0
(22) Date of filing: 14.03.2014
(51) Int. Cl.: G01N 33/50, G01N 33/68

(54) **A NONINVASIVE METHOD FOR MEASURING ANTIMICROBIAL PEPTIDES FROM SKIN AS AN OBJECTIVE MEASUREMENT OF NATURAL PROTECTION FROM MICROBES**
NICHTINVASIVES VERFAHREN ZUR MESSUNG ANTIMIKROBIELLER PEPTIDE AUS DER HAUT ALS OBJEKTIVE MESSUNG VON NATÜRLICHEM SCHUTZ GEGEN MIKROBEN
PROCÉDÉ NON INVASIF DE MESURE DE PEPTIDES ANTIMICROBIENS SUR LA PEAU EN TANT QUE MESURE OBJECTIVE DE LA PROTECTION NATURELLE À PARTIR DE MICROBES

(30) Priority: 15.03.2013 US 201361794014 P
(43) Date of publication of application: 28.02.2018
(62) Divisional of application: 14724207.7
(73) Proprietor: The Procter & Gamble Company, Cincinnati, OH 45202 (US)
(72) Inventor: KERR, Kathleen, Cincinnati, OH 45202 (US); FIENO, Angela, Cincinnati, OH 45202 (US); SUN, Yiping, Cincinnati, OH 45202 (US); CHAUDHARY, Tanuja, Cincinnati, OH 45202 (US); GRANT, Raymond, Cincinnati, OH 45202 (US); MILLS, Kevin, Cincinnati, OH 45202 (US)
(74) Representative: P&G Patent Germany

(56) References cited:
- WO-A1-2009/095456
- WO-A2-2004/056307
- US-A1- 2005 221 334
- Nobuhiko Eda ET AL: "Effects of High-Intensity Endurance Exercise on Epidermal Barriers against Microbial Invasion", Journal of sports science & medicine, 1 January 2013 (2013-01-01), pages 44-51, XP055132091, Turkey Retrieved from the Internet: URL:http://www.ncbi.nlm.nih.gov/pubmed/241 49724
- SCHLAPBACH C ET AL: "Human beta-defensin-2 and psoriasin are overexpressed in lesions of acne inversa", JOURNAL OF THE AMERICAN ACADEMY OF DERMATOLOGY, MOSBY, INC, US, vol. 61, no. 1, 1 July 2009 (2009-07-01), pages 58-65, XP026336023, ISSN: 0190-9622, DOI: 10.1016/J.JAAD.2008.12.033 [retrieved on 2009-02-26]
- T. GAMBICHLER ET AL: "Significant upregulation of antimicrobial peptides and proteins in lichen sclerosus", BRITISH JOURNAL OF DERMATOLOGY, vol. 161, no. 5, 1 November 2009 (2009-11-01), pages 1136-1142, XP055132136, ISSN: 0007-0963, DOI: 10.1111/j.1365-2133.2009.09273.x
- JENS-MICHAEL JENSEN ET AL: "Barrier Function, Epidermal Differentiation, and Human [beta]-Defensin 2 Expression in Tinea Corporis", JOURNAL OF INVESTIGATIVE DERMATOLOGY, 29 March 2007 (2007-03-29), XP055131632, ISSN: 0022-202X, DOI: 10.1038/sj.jid.5700788

## Description

### FIELD OF THE INVENTION

The present invention relates to a method for diagnosing dandruff based on the level of the antimicrobial peptide biomarker human beta defensin-2 (hBD2) and the link and correlation of the amount of these biomarkers as it directly correlates to improvement in dandruff in mammals.

### BACKGROUND OF THE INVENTION

The scalp/skin is a remarkable organ system composed of multiple specialized tissues that function as a first line of defense against environmental insults. While early research focused primarily on the barrier function of the skin, it has become clear that that this organ is dynamic: sensing and responding to even small changes in the environment in order to help maintain homeostasis. Environmental influences such as solar radiation, pollution, or even the application of skin care products, result in a complex cascade of events that ultimately lead to changes in the expression of hundreds or thousands of genes. These changes in gene expression are generally translated to changes in protein production (or accumulation, release, modification, etc.) that catalyze chemical reactions ultimately leading to the cellular response. As these chemical reactions proceed, metabolic byproducts are often left as an indicator of what chemical processes have taken place. Analysis of these resulting proteins and small molecule biomarkers which correlate to a given skin condition, for example, dandruff can provide valuable information in understanding the condition as well as developing products for the purpose of diagnose and/or improve the skin condition. Dandruff is a common chronic relapsing scalp skin condition with flaking and itching sensations. The pathogenesis of dandruff is complex, and appears to be the result of interactions among scalp skin, microflora and the host immune system. Much of the previous work on this condition has focused on the examination of a few surface-level phenomena. Traditional expert- and self-observation-based assessments are combined with largely instrumental-based assessments of epidermal structure and function at the physiological level. New biomolecular capabilities establish a depth of pathophysiological understanding not previously achievable with traditional means of investigation; however a clear picture of the molecular events leading to the key symptoms of this condition has yet to emerge. To elucidate these key molecular events biomolecular sampling can be obtained noninvasively by tape stripping of the skin surface followed by chemical or bioanalytical methodologies. Histamine was recently identified as a sensitive biomarker for scalp itch. Additional biomarkers are needed to enable a more detailed pathophysiological description of the dandruff condition as well as serve as relevant measures indicative of the extent and completeness of therapeutic resolution of dandruff. One such area of interest is antimicrobial peptides (AMPs).

Antimicrobial peptides (AMPs) are evolutionarily conserved molecules involved in the defense mechanisms of a wide range of organisms. Produced in bacteria, insects, plants and vertebrates, AMPs protect against a broad array of infectious agents. In mammals these peptides protect against bacteria, viruses, fungi, and certain parasites. Recently, novel biologic effects of AMPs have been documented such as endotoxin neutralization, chemotactic and immunomodulating activities, induction of angiogenesis and wound repair. Thus these molecules are crucial components of the innate immune system and attractive candidates for biomarkers. Specifically for skin and scalp, AMPs are important to skin and scalp health due to their ability to develop a chemical defense or barrier once the physical structure of the epidermis has been compromised. AMPs are expressed on the primary barriers of the organism such as skin and mucosal epithelia, and at sites of potential microbial entry in the skin such as follicular structures and sweat glands to prevent the colonization of host tissues by pathogens. Moreover, these peptides are stored in granules within phagocytes, where they assist in the killing of engulfed microorganisms. The mode of action by which antimicrobial peptides work is varied and includes disrupting membranes, interfering with metabolism, and targeting cytoplasmic components. The classification of AMPs is difficult owing to their considerable diversity. Based on their amino acid composition, size and conformational structures, AMPs can be divided into several categories, such as peptides with α-helix structures, peptides with β-sheet structures stabilized by disulfide bridges, peptides with extended structures, and peptides with loop structures. All together these AMPs' properties make these peptides attractive candidates for novel biomarkers.

Nobuhiko et al. (Journal of Sports Science and Medicine (2013) 12; 44-51) analyze effects of high-intensity endurance exercise on epidermal barriers against microbial invasion. In detail, samples from skin surface from athletes pre, during and post exercise were collected and analyzed for the concentration of SigA and hBD2 and the moisture content. For hBD2, it was found that the concentration of hBD2 increased during and post exercise.

Schlapbach et al. (Journal of the American Academy of Dermatology (2009) 61; 58-65) present the findings that human β-defensin-2 (hBD2) and psoriasin are overexpressed in lesions of acne inversa.

Gambichler et al. (British Journal of Dermatology (2009) 161, 5; 1136-1142) report significant upregulation of antimicrobial peptides and proteins in lichen sclerosus that is a chronic inflammatory T cell-driven sclerotic skin condition.

Jensen et al. (Journal of Investigative Dermatology (2007) 127, 1720-10727) is directed to barrier function, epidermal differentiation, and human β-defensin-2 expression in tinea corporis which is a superficial mycotic infection resulting in substantial epidermal changes.

International Patent Application No.: WO 2009/095456 A1 relates to peptide derivatives useful in the treatment, care or cleansing of the skin, mucosae, scalp or nails. The peptide derivatives are capable of stimulating the production of endogenous β-defensins.

International Patent Application No.: WO 2004/056307 A2 relates to methods of treating diseases using anti-antimicrobial peptide (AMP) and/or anti-AMP-like molecule (AML) inhibitors, to methods of treating diseases using AMPs/AMLs, and to methods of identifying compounds capable of decreasing activities/levels of AMPs/AMLs so as to enable treatment of diseases.

### SUMMARY OF THE INVENTION

The present invention is directed to a noninvasive method for measuring dandruff in a subject comprising collecting an epithelial cell /skin cell sample from the subject; detecting a level of the antimicrobial peptide biomarker human beta defensin-2 (hBD2) in the epithelial cell sample/skin cell sample; diagnosing the subject as having dandruff based on the level of the antimicrobial peptide biomarker human beta defensin-2 (hBD2), by determining the amount of the human beta defensin-2 (hBD2) biomarker in the epithelial cells as compared to a normal population.

### BRIEF DESCRIPTION OF THE DRAWINGS

Figure 1 is graph showing a comparison of hBD2 levels in non-dandruff subjects versus dandruff sufferers.
Figure 2 is a graph showing the change from baseline for hBD2 in dandruff sufferers after three weeks of treatment with an anti-dandruff shampoo or non-dandruff shampoo.
Figure 3 is a graph showing normalization of hBD2 in dandruff sufferers after three weeks of treatment with a 1% ZPT containing shampoo.
Table 1 is a list of relevant antimicrobial peptides.
Table 2 is a list of antimicrobial peptides detected by the iTRAQ methodology. Comparisons are shown between non-dandruff, dandruff sufferers at baseline and dandruff sufferers treated for 3 weeks with a 1% ZPT containing shampoo or a 1% selenium sulfide shampoo.

### DETAILED DESCRIPTION OF THE INVENTION

While the specification concludes with claims which particularly point out and distinctly claim the invention, it is believed the present invention will be better understood from the following description.

The present invention can comprise, consist of, or consist essentially of the essential elements and limitations of the invention described herein, as well any of the additional or optional ingredients, components, or limitations described herein.

All percentages, parts and ratios are based upon the total weight of the compositions of the present invention, unless otherwise specified. All such weights as they pertain to listed ingredients are based on the active level and, therefore; do not include carriers or by-products that may be included in commercially available materials.

The components and/or steps, including those, which may optionally be added, of the various embodiments of the present invention, are described in detail below.

All ratios are weight ratios unless specifically stated otherwise.

All temperatures are in degrees Celsius, unless specifically stated otherwise.

Except as otherwise noted, all amounts including quantities, percentages, portions, and proportions, are understood to be modified by the word "about", and amounts are not intended to indicate significant digits.

Except as otherwise noted, the articles "a", "an", and "the" mean "one or more"

Herein, "comprising" means that other steps and other ingredients which do not affect the end result can be added. This term encompasses the terms "consisting of' and "consisting essentially of'. The compositions and methods/processes of the present invention can comprise, consist of, and consist essentially of the essential elements and limitations of the invention described herein, as well as any of the additional or optional ingredients, components, steps, or limitations described herein.

Herein, "effective" means an amount of a subject active high enough to provide a significant positive modification of the condition to be treated. An effective amount of the subject active will vary with the particular condition being treated, the severity of the condition, the duration of the treatment, the nature of concurrent treatment, and like factors.
As used herein, the term "differential level" of a biomolecule may include any increased or decreased level. In one embodiment, differential level means a level that is increased by: at least 5%; by at least 10%; by at least 20%; by at least 30%; by at least 40%; by at least 50%; by at least 60%; by at least 70%; by at least 80%; by at least 90%; by at least 100%; by at least 110%; by at least 120%; by at least 130%; by at least 140%; by at least 150%; or more.

In another embodiment, differential level means a level that is decreased by: at least 5%; by at least 10%; by at least 20%; by at least 30%; by at least 40%; by at least 50%; by at least 60%; by at least 70%; by at least 80%; by at least 90%; by at least 100% (i.e., the biomolecule is absent). A biomolecule is expressed at a differential level that is statistically significant (i.e., a p-value of ≤ 0.20 (two-sided) was used to determine statistical significance) as determined using, either Student T-test, Welch's 2- sample T-test or Matched Pair T-test.

The term 'skin' means the outer covering of a vertebrate animal, consisting of two layers of cells, a thick inner layer (the dermis) and a thin outer layer (the epidermis). The epidermis is the external, nonvascular layer of the skin. It is made up, from within outward, of five layers of EPITHELIUM: (1) basal layer (stratum basale epidermidis); (2) spinous layer (stratum spinosum epidermidis); (3) granular layer (stratum granulosum epidermidis); (4) clear layer (stratum lucidum epidermidis); and (5) horny layer (stratum corneum epidermidis).

The term "sample" refers to any preparation from skin or epidermis of a subject.

The term "noninvasive" means a procedure that does not require insertion of an instrument or device through the skin or a body orifice for diagnosis or treatment.

The term "adhesive device" means a device used for the removal of the skin's epidermal layer by using an adhesive or an adhesive material on a substrate. For example, skin samples with adhesive tapes such as D-Squame® (polyacrylate ester adhesives; CuDerm; Dallas TX), Durapor, Sebutape™ (acrylic polymer films; CuDerm; Dallas, TX), Tegaderm™, Duct tape (333 Duct Tape, Nashua tape products), Scotch® Tape (3M Scotch 810, St. Paul, Minn.), Diamond™ (The Sellotape Company; Eindhoven, the Netherlands), Sentega™ (polypropylene tape, Sentega Eiketten BV, Utrecht, The Netherlands) may be used. The adhesive may be any of the commonly used pressure-sensitive-type adhesives or those which solidify quickly upon skin content (such as cynaoacylates). The adhesives may be on flexible or solid backings to make sampling easier. A constant pressure device (e.g. D-Squame Pressure Instrument, CuDerm; Dallas, TX) can be used to apply pressure to the adhesive device during sampling.

Samples from a tissue may be isolated by any number of means well known in the art. Invasive methods for isolating a sample include the use of needles, for example during blood sampling, as well as biopsies of various tissues, blistering techniques and laser poration. Due to the invasive nature of these techniques there is an increased risk of mortality and morbidity. Further, invasive techniques can inadvertently impact the state of the skin, which could lead to inaccurate or false results. Even further, invasive techniques are difficult to execute on a large population. The invasive technique may result in discomfort to the participant and may provide a greater potential for infection or other side effects. The present invention provides a noninvasive method for measuring antimicrobial peptides from the skin.

The term "objectively" means without bias or prejudice. Alternatively, any expert or self-assessments are inherently "subjective."

The term "normalization" and/or 'normalized" means the degree to which a population of dandruff sufferers approach a state of normal population.

The term "standardization" and/or "standardized" means antimicrobial protein values expressed relative to the amount of protein measured on the corresponding adhesive or adhesive article. A non-limiting example would be ng antimicrobial protein / µg soluble protein.

The term "baseline" means information gathered at the beginning of a study from which variations found in the study are measured. A baseline sample may be from a dandruff sufferer or a non-dandruff subject.

In a further embodiment of the present invention, there are a number of Alternative "Noninvasive" Sampling Methods that may be used.

Sebutape™: This is a noninvasive approach in that Sebutape™ (acrylic polymer film; CuDerm; Dallas, TX) is only very mildly adhesive and may be applied to and removed from even visibly inflamed skin without causing discomfort. Biomarkers recovered/assayed by this technique have included proteins (e.g., cytokines), peptides (e.g., neuropeptides), and mediators. Historically, this tape is manufactured and sold for sebum collection and can, therefore, be useful for lipid analysis.

D-Squame®: D-Squame® tape is a polyacrylate ester adhesive also manufactured by CuDerm. It may be used to recover the same biomarkers as Sebutape™ but also removes certain epidermal structural proteins (e.g., keratins, involucrin). It has also been used to recover cortisol and serum albumin as systemic inflammatory markers, and stratum corneum lipids.

Cup Scrubs: Cup scrubs extract proteins directly from the surface of the skin, usually in the presence of buffer and a nonionic surfactant. Cup scrubs are primarily used for recovery of soluble biomarkers such as cytokines, but can also be used to recover small organic molecules. Many more cytokines can be recovered and quantified from cup scrubs than from tape strips. This could be due to several reasons. (a) Due to the presence of detergents and their liquid nature, cup scrubs most likely sample a different protein population than do tape strips. (b) With cup scrubs, cytokines do not have to be further extracted after sample collection since they already are in solution.

Hair plucks: Plucking hairs is the process of removing human or animal hair by mechanically pulling the item from the owner's body usually with tweezers. The follicular region of the hair pluck is extracted usually in the presence of buffer and a nonionic surfactant for recovery of soluble biomarkers such as cytokines, and can also be extracted with an organic solvent to recover small organic molecules.

Animal (i.e. Dog) Collection Method: D-Squame®: D- Squame™ tape samples are collected on dogs' skin via parting their fur (without shaving). A variety of biomarkers related to skin inflammation, differentiation and barrier integrity can be analyzed from the tapes including total protein, soluble protein, skin multiple analyte profile (skin MAP), skin cytokines and stratum corneum lipids (ceramides, cholesterol, fatty acids).

In an embodiment of the present invention, the present invention provides a method and analysis for noninvasively obtaining a sample for use in isolating antimicrobial proteins.

In an embodiment, the use of an adhesive device can be used to achieve such sampling.
In preparation for such a sampling study for a dandruff sampling, at a baseline visit, a qualified screening grader will complete adherent scalp flaking score (ASFS) grading for each subject and the highest flaking octant will be identified for tape strip sampling. The highest flaking octant will be sampled at baseline and after a treatment period. Tape strips samples will be collected from each subject at each time point (e.g., baseline and week 3).

The tape strip sampling is repeated additional times, as needed, at the same site placing each D-Squame® tape disc on top of the prior sampled area. The D-Squame® tapes after sample collection are placed into the appropriately labeled wells in a labeled plate.

Following the sampling, an extraction and quantitation procedure is conducted. In an embodiment of the present invention, quantitation of antimicrobial protein from extracts of D-Squame® Tape Samples can be conducted via analysis by either iTRAQ protein profiling or an antibody-based assay.

iTRAQ (**i**sobaric **T**ag for **R**elative and **A**bsolute **Q**uantitation) is a non-gel based proteomics approach, originally developed by ABSciex. It can be used for protein identification (http://en.wikipedia.org/wiki/Protein) and relative quantitation simultaneously from samples under different biological treatments/conditions in one single experiment. The technique uses 4- or 8-isotope-coded covalent tags varying mass to label the N-terminus and side-chain amine of the peptides from protein digestion. After the labeling of all peptides from different samples/treatments, samples are pooled and analyzed by liquid chromatography-tandem mass spectrometry like LC-MALDI-TOF/TOF MS.
In this embodiment of the present invention, the sample extraction in preparation for antimicrobial peptide analysis via iTRAQ was performed.

The D-Squame® Tape Samples plates are removed from -80°C freezer where they are stored following sample collection, and placed on dry ice. The tape strips are inserted into pre-labeled polypropylene collection tubes, adhesive side facing inward.

Extraction buffer containing 5% acetic acid is added to each collection tube and then extracted on ice using sonication. If necessary, additional tapes are placed in the collection tube containing the extract solution and the extraction process is repeated as a means of concentrating the sample. Each extract solution is isolated from the tape strip and an aliquot of each sample is placed into a pre-labeled polypropylene collection tube and frozen at -80°C for analysis. A separate aliquot is specified for soluble protein analysis using a BCA™ Protein Assay Kit, Pierce catalog #23227.

Following the extraction process, iTRAQ Labeling-iTRAQ assays are performed by following manufacture recommended protocol. Briefly, equal amount of proteins about 10-50ug from each condition are reduced, alkylated and digested with enzyme trypsin at 37C overnight. Each digest is labeled with a specific iTRAQ reagent (e.g., reagent 113, 114, etc) and then mixed together to produce a single sample mixture for further protein identification and quantitation using mass spectrometry.

In an embodiment of the present invention, the multiplexed iTRAQ labeled samples are subjected to low flow LC separation at 1-5ul/min using Tempo LC-MALDI spotter system (ABSciex) or Waters CapLC-Proteineer Spotter (Bruker Daltonics). Peptides eluted off the column (0.1mm x 15cm monolithic silica column from Merck) are mixed continuously with MALDI matrix (α-Cyano-4-hydroxycinnamic acid -CHCA) and spotted automatically onto a MALDI plate. MALDI-TOF/TOF analysis is performed either on AB Sciex MALDI AB4800 *Plus* or Bruker UltrafleXtreme MALDI-TOF/TOF system. MS and MS/MS data are collected. Protein identification and relative quantitation are done either by ProteinPilot software (AB Sciex) or Mascot (Matrix Science) and WarpLC software (Burker Daltonics). For protein identification, the data sets are searched against Uniprot for the MALDI AB4800 *Plus* data and NCBInr for the UltrafleXtreme MALDI data. For relative quantitation, the signals of the iTRAQ reporter ions of MS/MS spectra are used for calculating the relative abundance (ratio) of the peptides identified. The combined ratios of the peptides from a specific protein represent the relative quantification of that protein. All of the mass spectrometry data is initially processed automatically using vendors' software package without any modifications. The mass spectrometry data is further processed using Statistical Analysis System (SAS Institute Inc.) for calculating the iTRAQ ratio for relative quantitation of proteins (Table 2). The relative quantitated values for the compounds are then adjusted according to sample volume and standardized to the amount of soluble protein in the extract as determined by the BCA protein assay as outlined above.

In this embodiment of the present invention, the sample extraction in preparation for antimicrobial peptide analysis via antibody-based assay is performed.

For the antibody-based immunoassay, appropriate standard extraction buffers are added to each collection tube and then extracted on ice using sonication. Each extract solution is isolated from the tape strip and an aliquot of each sample is placed into a specified position of a 96-well polypropylene plate. Aliquots of the extracts of D-Squame® Tape samples are then supplemented with conventional reagents, such as albumin, to help prevent loss of analytes to the walls of labware, transferred into 96-well polypropylene deep well plates and frozen at -80°C for analysis. A separate aliquot is not supplemented with reagents and is analyzed for soluble protein using a BCA™ Protein Assay Kit, Pierce catalog #23227.

Following the extraction process, appropriate standards and controls can be prepared by conventional methods. AMPs will be quantitated with an immunoassay on the Mesoscale Discovery platform. The result can be reported as the amount of AMP/tape strip or the result can be standardized by dividing by the amount of AMP by the amount of the protein that was also found in the tape strip extract. The protein method has been described separately. Data analysis is conducted by standard statistical methods and calculations.

### Methodology Extension

Although the exact procedure used is described above, there are a number of alternate approaches that could be taken for a number of the steps outlined above that are logical extensions. The extraction solvent employed for isolating AMPs means antimicrobial protein from the tape strip can be any appropriate aqueous, organic or organic/aqueous mixture that provides a suitable recovery. LC/MS/MS and GC/MS/MS are generally recognized as state-of-the-art approaches for the quantitative analysis of organic molecules in biological matrices due to its high selectivity and sensitivity. However, any analytical technique and or other approach providing the required sensitivity and selectivity could be employed, e.g., iTRAQ labeling followed by Liquid Chromatography-Electrospray Ionization Mass Spectrometry, Label-Free Quantitation Mass Spectrometry and Multiple Reaction Monitoring (MRM) Mass Spectrometry. For example, other methods for assessing AMPs have been employed including: NMR, capillary electrophoresis, supercritical fluid and other chromatographic techniques and/or combinations thereof. Similarly, instrumental approaches without separation techniques have also been employed including mass spectrometry, electrochemical and fluorometric assays. Additionally, ligand binding approaches such as competitive and non-competitive enzyme linked immunosorbent assays (ELISAs) and radioimmunoassay (RIA) or other labeling schemes have also been employed. Enzyme-based and anti-body-based assays have a long history of use in the analysis of like AMPs. Bioassay using either cell-based or tissue based approaches could have also been used as the means of detection. In an embodiment of the present invention, quantitation of antimicrobial proteins from hair plucks can be carried out with the same basic extraction and analysis methods as used for tape strip samples.

### Protein Determination of Tape Strip Extracts:

The level of AMP on tape strip samples of skin measured using a suitable methodology described above can be standardized using amount of protein found in the tape strip extract. Standardization is done by dividing the antimicrobial protein level by the amount of protein in the tape strip extract.

The amount of protein in the tape strip extract or an equivalent matrix that is used to determine the antimicrobial peptide level on skin can be determined using variety of protein determination methods described in the literature. Examples of such methods include total nitrogen determination, total amino acid determination and protein determination based on any colorimetric, flurometric, luminometric methods. These methods may or may not involve further sample preparation of the tape strip extract prior to protein determination. A non-limiting example of a specific method for protein determination in the tape strip extract is given below. A comprehensive review of protein determination methods, their applicability and limitations are described in the Thermo Scientific Pierce Protein Assay Technical Handbook that can be downloaded from the following link. www.piercenet.com/Files/1601669_PAssayFINAL_Intl.pdf. Further information related to protein determination can be found at Redinbaugh, M.G. and Turley, R.B. (1986). Adaptation of the bicinchoninic acid protein assay for use with microtiter plates and sucrose gradient fractions. Anal.Biochem. 153, 267-271.

Adhesive tapes sampled from human skin will be extracted and analyzed for protein content using the BCA™ Protein Assay Kit (Pierce). The tape strips sampled from human skin will be extracted with a conventional extraction buffer. Following extraction, aliquots of the tape extracts will be transferred into 96-well polypropylene deep well plates and stored at 2 - 8°C for protein determination.

The BCA™ Protein Assay Kit is based on the reduction of Cu²⁺ to Cu¹⁺ by proteins in an alkaline medium coupled with the sensitive and selective colorimetric detection of Cu⁺¹ by bicinchoninic acid (BCA). The purple-colored reaction product, formed by chelation of two molecules of BCA with one Cu¹⁺ ion, exhibits strong absorbance at a wavelength of 562 nm. The optical density (OD) is measured using a microplate reader. Increasing concentrations of Bovine Serum Albumin (BSA), expressed in micrograms per milliliter (µg/mL), are used to generate a calibration curve in the assay. Appropriate assay QC's prepared from the BSA stock solution will be used to monitor assay performance during sample analysis.

In an alternative embodiment of the present invention, protein determination can be done direct measurement of protein on an adhesive or an adhesive article such as protein measurement with a SquameScan® 850A (CuDerm Corporation, Dallas, Texas).

### EXAMPLES

### Basic Procedure for AMP analysis

Subjects are evaluated by a qualified grader to establish their scalp status. Dandruff subjects are identified by their level of visible flaking as assessed by a qualified grader. Non-dandruff healthy scalp subjects are evaluated at baseline to establish normalized levels of each biomarker. Subjects are identified by a qualified grader in two separate clinical studies; study #1 and study #2. In Study #1, there are 60 non-dandruff subjects that were evaluated at baseline for hBD2 levels. There are 119 dandruff sufferers placed on a 1% ZPT containing anti-dandruff shampoo, 115 dandruff sufferers placed on a 1% selenium sulfide containing anti-dandruff shampoo and 60 dandruff subjects placed on a non-dandruff shampoo that does not contain an anti-dandruff active. The subjects are evaluated for human beta defensin 2 (hBD2) levels at baseline, and after 3 weeks of product usage.

In study #2, there are 121 non-dandruff subjects, 311 dandruff subjects are placed on a 1% ZPT containing shampoo and 63 dandruff subjects are placed on a 1% Selenium sulfide containing shampoo. Tape strip samples for each group are analyzed using the iTRAQ methodology.

The dandruff subjects undergo a three week treatment period with an anti-dandruff shampoo (a shampoo composition containing 1% zinc pyrithione or 1% selenium sulfide or no anti-dandruff active), tape strip samples are collected from the highest flaking octant as determined at the baseline visit by qualified grader. For dandruff subjects, scalp tape strips are taken at baseline and after a three week product treatment. For non-dandruff subjects, scalp tape strips are taken at baseline only. Tapes are kept at -80°C until extracted. A dandruff-involved site is sampled by parting the hair, applying a D-Squame® tape (CuDerm Corporation), and rubbing the tape, as needed. For the non-dandruff subjects, a flake free site is sampled. The tapes are placed into a pre-labeled 12 well culture dish for storage.

Samples are extracted with a conventional extraction buffer and sonicated on ice. Aliquots of the extracts of D-Squame® Tape samples are then transferred into pre-labeled polypropylene collection tubes and frozen at -80°C for analysis. A separate aliquot is specified for soluble protein analysis using a BCA™ Protein Assay Kit, Pierce catalog #23227

Following extraction, the samples are analyzed for protein expression profiling by iTRAQ analysis or immunoassay based method. The relative quantitated values for the compounds are standardized to the amount of soluble protein in the extract as determined by the BCA protein assay as outlined above. Analysis is conducted at baseline and week 3 samples from the anti-dandruff shampoo treatment group and for baseline only for the non-dandruff group. AMPs are detected via iTRAQ, and identified based on matched known chemical structures in Uniprot and NCBInr databases. AMPs are quantified by immunobased assay using appropriate standards and controls with an immunoassay on the Mesoscale Discovery platform which are specifically designed for each individual AMP. Matched pair T-test is used to analyze the differences among the non-dandruff (healthy), dandruff baseline and dandruff treated subjects. Dandruff treated subjects are compared to non-dandruff to determine the degree of normalization upon treatment with an anti-dandruff product.

### RESULTS SUMMARY

Statistical Analysis of Data: Data are collected on dandruff subjects at both baseline and at week 3 (after treatment) and non-dandruff subjects at baseline. Given the wide variability of analyte levels between subjects as well as within subject (baseline -> week 3), it is more efficient to transform the data to the log10 scale before analysis. Statistical analysis is carried out on the equivalent log10 Ratio values (i.e., log10(Week 3/Baseline)). Final reported results are then converted back to their original scale (or % change from baseline values), for ease of interpretation. All statistical analyses are carried out using the SAS JMP (Version 7.0.2) software. A p-value of ≤ 0.20 (two-sided) is used to determine statistical significance.

Relevant antimicrobial peptides are shown in Table 1. Table 1 contains 84 known antimicrobial peptides categorized and reviewed via UniProt. The non-redundant UniProt Entry name along with commonly used protein names are listed. The 11 proteins identify by iTRAQ in this study are also shown in Table 1. Seven out of eleven proteins identified by iTRAQ match with the known AMPs. They are: P81534 (HBD3), P59666 (HNP-3), P81605 (Dermcidin), P05109 (S100A8), P06702 (S100A9), P08311 (cathepsin G), and Q9H1E1 (RNase 7). The antimicrobial proteins identified by iTRAQ cover the majority of the categorized AMPs.

Table 2 lists 11 proteins identified by iTRAQ in this study having at least one statistical significant difference between the groups. Although several of these proteins in Table 2 have not yet been officially categorized as AMPs, there are a number of literature references indicating that these proteins function as AMPs (Lee DY et al., Histone H4 is a major component of the antimicrobial action of human sebocytes J Invest Dermatol. 2009 Oct;129(10):2489-96. doi: 10.1038/jid.2009.106. Epub 2009 Jun 18; Pavia, KE et al. Novel Histone-Derived Antimicrobial Peptides Use Different Antimicrobial Mechanisms. Biochimica et Biophysica Acta (BBA)-Biomembranes Volume 1818, Issue 3, March 2012, Pages 869-876; Gläser, R. et al. Antimicrobial psoriasin (S100A7) protects human skin from Escherichia coli infection. Nature Immunology 6, 57 - 64 (2004). Published online: 28 November 2004; | doi:10.1038/ni1142.

In addition to the reported antimicrobial function, their molecular structure is similar to some known AMP families (e.g., Histone and S100 proteins). The data in Table 2 shows the ratios and associated p-values for antimicrobial peptides identified by iTRAQ methodology for non-dandruff and dandruff subjects at baseline and for dandruff subjects treated with either 1% ZPT containing or 1% selenium sulfide containing shampoo after 3 weeks of treatment versus baseline. Additionally, a comparison of treated dandruff subjects versus non-dandruff subjects is shown as an indication of normalization to a healthy state.

The following antimicrobial peptides are identified: Histone H2B type 1-M, Histone H2A, Histone H4, Protein S100-A7, Protein S100-A8, Protein S100-A9, Cathepsin G, Neutrophil defensin 3 (Defensin, alpha 3) (HNP-3), Dermcidin, Ribonuclease 7 (RNase 7), and Beta-defensin 103 (hBD-3). As demonstrated by a ratio >1, all of the AMPs are elevated in dandruff relative to non-dandruff at baseline except for dermcidin which is lower in dandruff versus non-dandruff. After treatment with an anti-dandruff shampoo containing 1% ZPT or 1% selenium sulfide the ratios are decreased for all of the AMPs that are elevated in dandruff indicating an improvement. Dermcidin is increased versus baseline indicating an improvement. When the dandruff treated subject levels are compared to the non-dandruff subjects many of the AMPs are returning to levels associated with healthy scalp as indicated by ratio of ≤1 (or ≥1 for dermcidin).

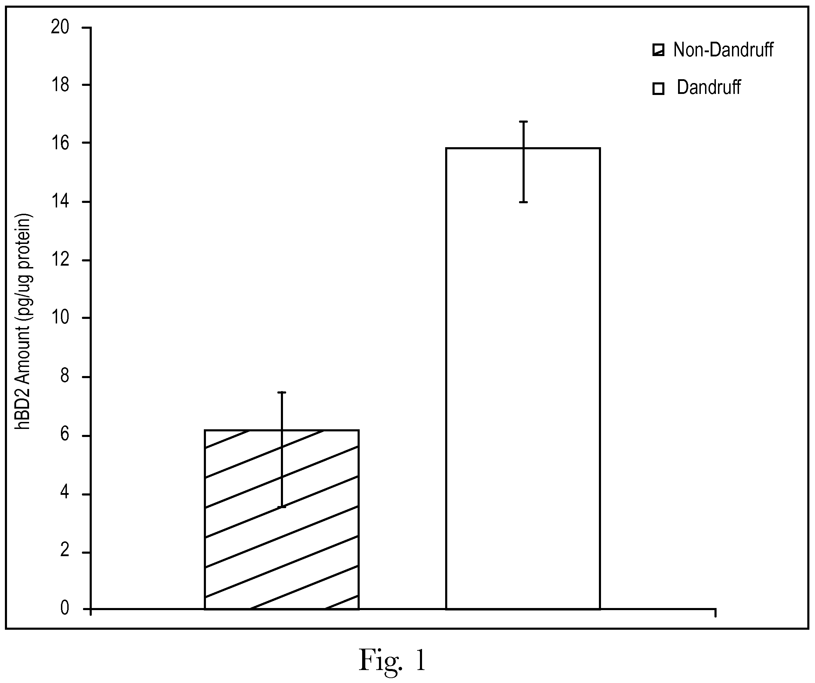

The data in Figure 1 demonstrates that Dandruff hBD2 levels are significantly higher than non-dandruff levels (p-value = <0.0001). These hBD2 levels are divided by the amount of protein in the tape strip extract. hBD2 levels are determined from an independent non-dandruff group to establish normal skin hBD2 levels.

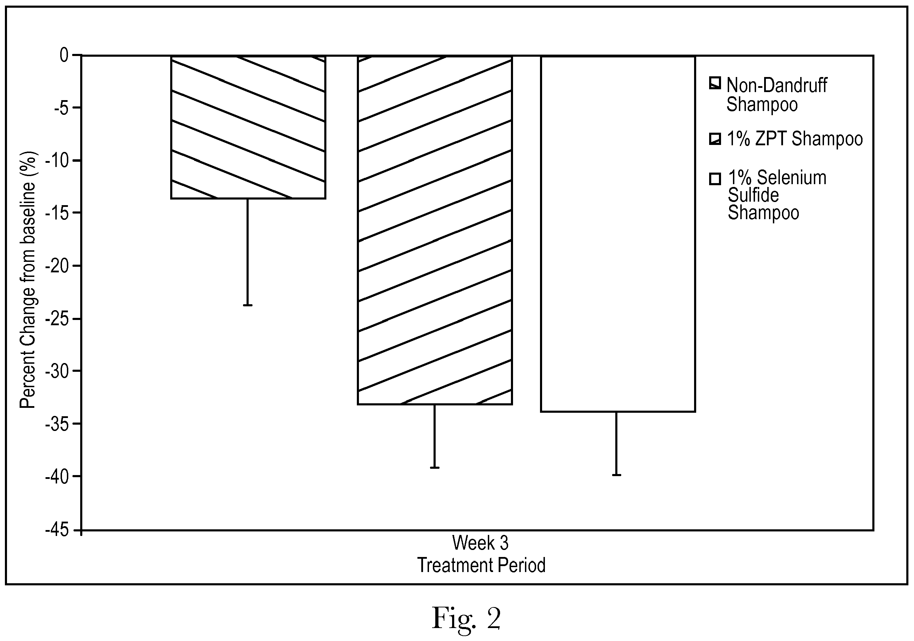

The data in Figure 2 demonstrates that there is a 33% reduction in hBD2 level following treatment with an anti-dandruff shampoo over a 3-week period of time versus baseline levels (p-values <0.0001). A reduction in hBD2 is consistent with improvement as demonstrated by comparing levels to the non-dandruff population. The anti-dandruff shampoo demonstrates a significant improvement versus a non-dandruff shampoo (p-value =0.046). hBD2 levels on tape strip samples of human scalp measured using a suitable methodology described above can be standardized using amount of protein found in the tape strip extract. These hBD2 levels have been divided by the amount of protein in the tape strip extract.

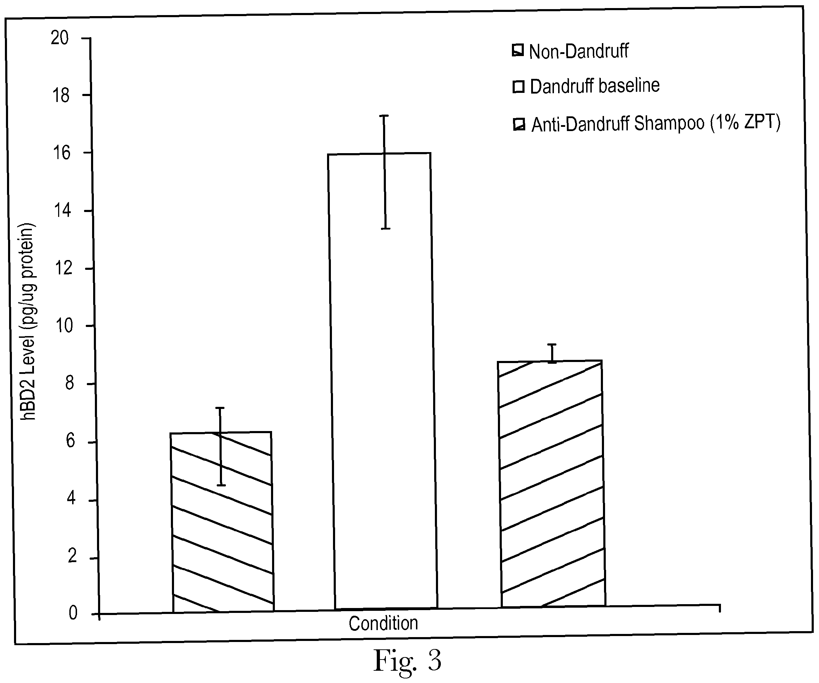

The data in Figure 3 demonstrates that the anti-dandruff treatment is reducing hBd2 levels more consistent to that of the non-dandruff group as discussed in Figure 1 above. This is a useful means of communicating the benefit as the dandruff population desires a return to a value consistent with a normalized and/or healthy state.

In particular embodiments, such performance assessments can be advantageous for comparing a subject's skin health status before and after treatment with an anti-dandruff composition. For example, some anti-dandruff compositions may promote a faster speed of relief that will be experienced by the subject if the subject switches to and maintains a certain anti-dandruff composition or regimen. An assessment that allows objective measurement of particular skin health benefits of the subject could allow for the comparing of an subject's status before the treatment with a particular anti-dandruff composition or regimen and after the subject switches to the particular anti-dandruff composition or regimen. Additionally, such an assessment could be used as a supporting credentialing tool that supports particular skin health benefit claims that the particular treatment or regimen is promoting. For example, if a particular composition or regimen is promoting that it will decrease the symptoms of itch, an assessment as disclosed herein can be used to support the specific health benefit claim to show that the subject's discomfort from itch has decreased. Non-limiting elements of skin health that could be benefited by anti-dandruff compositions and regimens include itch, flaking, irritation, skin barrier integrity, dryness, oxidative stress and oxidative damage, self-defense, natural protection.

A further embodiment includes wherein there is a change in standardized biomarker following application with a zinc pyrithione shampoo when compared to a baseline level of biomarker prior to the application. In another embodiment, there is a change in standardized molecule biomarker following application with a selenium sulfide shampoo when compared to a baseline level of biomarker prior to the application. In a further embodiment, there may be a measure of biomarker that establishes an improvement of at least 5% in skin health compared to a normal population following treatment. In a further embodiment, there may be an improvement of at least 10%, at least 20%, at least 30%, at least 40%, at least 50%, at least 60%, at least 70%, at least 80%, at least 90% and further, 100% improvement in skin health compared to a normal population following treatment. In another embodiment, there may be at least a 5% difference between dandruff and non-dandruff (no treatment). Further, there may be at least 10% difference between dandruff and non-dandruff, at least 20%, at least 30%, at least 40%, at least 50%, at least 60%, at least 70%, at least 80%, at least 90% between dandruff and non-dandruff and further, 100% difference or greater between dandruff and non-dandruff.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Table 1**

| **Protein UniProt Entry** | **Identified in iTRAQ Study** | **AMP Family** | **Protein names** **Figure 1** **Table 1** |
|---|---|---|---|
| P17213 | | BPI | Bactericidal permeability-increasing protein (BPI) (CAP 57) |
| Q8N4F0 | | BPI | BPI fold-containing family B member 2 (Bactericidal/permeability-increasing protein-like 1) (BPI-like 1) (Long palate, lung and nasal epithelium carcinoma-associated protein 2) (RYSR) |
| P59826 | | BPI | BPI fold-containing family B member 3 (Ligand-binding protein RYA3) (Long palate, lung and nasal epithelium carcinoma-associated protein 3) |
| P59827 | | BPI | BPI fold-containing family B member 4 (Ligand-binding protein RY2G5) (Long palate, lung and nasal epithelium carcinoma-associated protein 4) |
| P60022 | | Defensin | Beta-defensin 1 (BD-1) (hBD-1) (Defensin, beta 1) |
| P81534 | P81534 | Defensin | Beta-defensin 103 (Beta-defensin 3) (BD-3) (DEFB-3) (HBD3) (hBD-3) (Defensin, beta 103) (Defensin-like protein) |
| Q8WTQ1 | | Defensin | Beta-defensin 104 (Beta-defensin 4) (BD-4) (DEFB-4) (hBD-4) (Defensin, beta 104) |
| Q8NG35 | | Defensin | Beta-defensin 105 (Beta-defensin 5) (BD-5) (DEFB-5) (Defensin, beta 105) |
| Q8N104 | | Defensin | Beta-defensin 106 (Beta-defensin 6) (BD-6) (DEFB-6) (Defensin, beta 106) |
| Q8IZN7 | | Defensin | Beta-defensin 107 (Beta-defensin 7) (BD-7) (DEFB-7) (Defensin, beta 107) |
| Q8NET1 | | Defensin | Beta-defensin 108B (Beta-defensin 8) (BD-8) (DEFB-8) (hBD-8) (Defensin, beta 108) (Defensin, beta 108B) |
| A6NDD2 | | Defensin | Beta-defensin 108B-like |
| Q30KR1 | | Defensin | Beta-defensin 109 (Defensin, beta 109) (Defensin, beta 109, pseudogene 1/1B) |
| Q30KQ9 | | Defensin | Beta-defensin 110 (Beta-defensin 10) (DEFB-10) (Beta-defensin 11) (DEFB-11) (Beta-defensin 111) (Defensin, beta 110) (Defensin, beta 111) |
| Q30KQ8 | | Defensin | Beta-defensin 112 (Beta-defensin 12) (DEFB-12) (Defensin, beta 112) |
| Q30KQ7 | | Defensin | Beta-defensin 113 (Beta-defensin 13) (DEFB-13) (Defensin, beta 113) |
| Q30KQ6 | | Defensin | Beta-defensin 114 (Beta-defensin 14) (DEFB-14) (Defensin, beta 114) |
| Q30KQ5 | | Defensin | Beta-defensin 115 (Beta-defensin 15) (DEFB-15) (Defensin, beta 115) |
| Q30KQ4 | | Defensin | Beta-defensin 116 (Beta-defensin 16) (DEFB-16) (Defensin, beta 116) |
| Q96PH6 | | Defensin | Beta-defensin 118 (Beta-defensin 18) (DEFB-18) (Defensin, beta 118) (Epididymal secretory protein 13.6) (ESP13.6) |
| Q8N690 | | Defensin | Beta-defensin 119 (Beta-defensin 120) (Beta-defensin 19) (DEFB-19) (Beta-defensin 20) (DEFB-20) (Defensin, beta 119) (Defensin, beta 120) (ESC42-RELA) |
| Q5J5C9 | | Defensin | Beta-defensin 121 (Beta-defensin 21) (DEFB-21) (Defensin, beta 121) |
| Q8N688 | | Defensin | Beta-defensin 123 (Beta-defensin 23) (DEFB-23) (Defensin, beta 123) |
| Q8NES8 | | Defensin | Beta-defensin 124 (Beta-defensin 24) (DEFB-24) (Defensin, beta 124) |
| Q8N687 | | Defensin | Beta-defensin 125 (Beta-defensin 25) (DEFB-25) (Defensin, beta 125) |
| Q9BYW3 | | Defensin | Beta-defensin 126 (Beta-defensin 26) (DEFB-26) (Defensin, beta 126) (Epididymal secretory protein 13.2) (ESP13.2) |
| Q9H1M4 | | Defensin | Beta-defensin 127 (Beta-defensin 27) (DEFB-27) (Defensin, beta 127) |
| Q7Z7B8 | | Defensin | Beta-defensin 128 (Beta-defensin 28) (DEFB-28) (Defensin, beta 128) |
| Q9H1M3 | | Defensin | Beta-defensin 129 (Beta-defensin 29) (DEFB-29) (Defensin, beta 129) |
| Q30KQ2 | | Defensin | Beta-defensin 130 (Beta-defensin 30) (DEFB-30) (Defensin, beta 130) |
| P59861 | | Defensin | Beta-defensin 131 (Beta-defensin 31) (DEFB-31) (Defensin, beta 131) |
| Q7Z7B7 | | Defensin | Beta-defensin 132 (Beta-defensin 32) (BD-32) (DEFB-32) (Defensin HEL-75) (Defensin, beta 132) |
| Q30KQ1 | | Defensin | Beta-defensin 133 (Defensin, beta 133) |
| Q4QY38 | | Defensin | Beta-defensin 134 (Defensin, beta 134) |
| Q30KP9 | | Defensin | Beta-defensin 135 (Defensin, beta 135) |
| Q30KP8 | | Defensin | Beta-defensin 136 (Defensin, beta 136) |
| 015263 | | Defensin | Beta-defensin 4A (Beta-defensin 2) (BD-2) (hBD-2) (Defensin, beta 2) (Skin-antimicrobial peptide 1) (SAP1) |
| Q01523 | | Defensin | Defensin-5 (Defensin, alpha 5) (HD5(20-94)) [Cleaved into: HD5(23-94); HD5(29-94); HD5(56-94); HD5(63-94)] |
| Q01524 | | Defensin | Defensin-6 (Defensin, alpha 6) |
| A8MXU0 | | Defensin | Putative beta-defensin 108A (Defensin, beta 108A) (Putative beta-defensin 108B pseudogene 1/2) |
| P59665 | | Defensin | Neutrophil defensin 1 (Defensin, alpha 1) (HNP-1) (HP-1) (HP1) [Cleaved into: HP 1-56; Neutrophil defensin 2 (HNP-2) (HP-2) (HP2)] |
| P59666 | P59666 | Defensin | Neutrophil defensin 3 (Defensin, alpha 3) (HNP-3) (HP-3) (HP3) [Cleaved into: HP 3-56; Neutrophil defensin 2 (HNP-2) (HP-2) (HP2)] |
| P12838 | | Defensin | Neutrophil defensin 4 (Defensin, alpha 4) (HNP-4) (HP-4) |
| P62807 | | Histone | Histone H2B type 1-C/E/F/G/I (Histone H2B.1 A) (Histone H2B.a) (H2B/a) (Histone H2B.g) (H2B/g) (Histone H2B.h) (H2B/h) (Histone H2B.k) (H2B/k) (Histone H2B.I) (H2B/I) |
| P06899 | | Histone | Histone H2B type 1-J (Histone H2B.1) (Histone H2B.r) (H2B/r) |
| O60814 | | Histone | Histone H2B type 1-K (H2B K) (HIRA-interacting protein 1) |
| Q16778 | | Histone | Histone H2B type 2-E (Histone H2B-GL105) (Histone H2B.q) (H2B/q) |
| P57053 | | Histone | Histone H2B type F-S (Histone H2B.s) (H2B/s) |
| | Q99879# | Histone | HistoneH2B type 1-M,histone cluster 1, H2bm, H2B histone family, member E H2BF, histone 1, H2bm, H2B, Histone H2B. dJ160A22.3, histone H2B type 1-M |
| | P16104# | Histone | HistoneH2A, Histone H2A.x, H2AX, H2AFX |
| | P62805# | Histone | Histone 4. HIST2H4A, HIST2H4B, |
| P15515 | | Pore-forming toxin | Histatin-1 (Histidine-rich protein 1) (Post-PB protein) (PPB) [Cleaved into: His1-(31-57)-peptide (His1 31/57) (His1-(12-38)-peptide) (His1 12/38) (Histatin-2)] |
| P15516 | | Pore-forming toxin | Histatin-3 (Basic histidine-rich protein) (Hst) (Histidine-rich protein 3) (PB) [Cleaved into: Histatin-3; His3-(20-44)-peptide (His3 20/44) (His3-(1-25)-peptide) (His3 1/25) (Histatin-3 1/25) (Histatin-6); His3-(20-43)-peptide (His3 20/43) (His3-(1-24)-peptide) (His3 1/24) (Histatin-3 1/24) (Histatin-5); His3-(20-32)-peptide (His3 20/32) (His3-(1-13)-peptide) (His3 1/13) (Histatin-3 1/13); His3-(20-31)-peptide (His3 20/31) (His3-(1-12)-peptide) (His3 1/12) (Histatin-3 1/12); His3-(20-30)-peptide (His3 20/30) (His3-(1-11)-peptide) (His3 1/11) (Histatin-3 1/11); His3-(24-32)-peptide (His3 24/32) (His3-(5-13)-peptide) (His3 5/13) (Histatin-3 5/13); His3-(24-31)-peptide (His3 24/31) (His3-(5-12)-peptide) (His3 5/12) (Histatin-11) (Histatin-3 5/12); His3-(24-30)-peptide (His3 24/30)(His3-(5-11)-peptide) (His3 5/11) (Histatin-12) (Histatin-3 5/11); His3-(25-32)-peptide (His3 25/32) (His3-(6-13)-peptide) (His3 6/13) (Histatin-3 6/13); His3-(25-30)-peptide (His3 25/30) (His3-(6-11)-peptide) (His3 6/11) (Histatin-3 6/11); His3-(26-32)-peptide (His3 26/32) (His3-(7-13)-peptide) (His3 7/13) (Histatin-3 7/13); His3-(26-31 )-peptide (His3 26/31) (His3-(7-12)-peptide) (His3 7/12) (Histatin-3 7/12); His3-(26-30)-peptide (His3 26/30) (His3-(7-11)-peptide) (His3 7/11) (Histatin-3 7/11); His3-(31-51)-peptide (His3 31/51) (His3-(12-32)-peptide) (His3 12/32) (Histatin-3 12/32) (Histatin-4); His3-(31-44)-peptide (His3 31/44) (His3-(12-25)-peptide) (His3 12/25) (Histatin-3 12/25) (Histatin-9); His3-(31-43)-peptide (His3 31/43) (His3-(12-24)-peptide) (His3 12/24) (Histatin-3 12/24) (Histatin-7); His3-(32-44)-peptide (His3 32/44) (His3-(13-25)-peptide) (His3 13/25) (Histatin-10) (Histatin-3 13/25); His3-(32-43)-peptide (His3 32-43) (His3-(13-24)-peptide) (His3 13/24) (Histatin-3 13/24) (Histatin-8); His3-(33-44)-peptide (His3 33/44) (His3-(14-25)-peptide) (His3 14/25) (Histatin-3 14/25); His3-(33-43)-peptide (His3 33/43) (His3-(14-24)-peptide) (His3 14/24) (Histatin-3 14/24); His3-(34-44)-peptide (His3 34/44) (His3-(15-25)-peptide) (His3 15/25) (Histatin-3 15/25); His3-(34-43)-peptide (His3 34/43) (His3-(15-24)-peptide) (His3 15/24) (Histatin-3 15/24); His3-(45-51 )-peptide (His3 45/51) (His3-(26-32)-peptide) (His3 26/32) (Histatin-3 26/32); His3-(47-51)-peptide (His3 47/51) (His3-(28-32)-peptide) (His3 28/32) (Histatin-3 28/32); His3-(48-51 )-peptide (His3 48/51) (His3-(29-32)-peptide) (His3 29/32) (Histatin-3 29/32)] |
| P81605 | P81605 | Pore-forming toxin | Dermcidin (EC 3.4.-.-) (Preproteolysin) [Cleaved into: Survival-promoting peptide; DCD-1] |
| P49913 | | Pore-forming toxin | Cathelicidin antimicrobial peptide (18 kDa cationic antimicrobial protein) (CAP-18) (hCAP-18) [Cleaved into: Antibacterial protein FALL-39 (FALL-39 peptide antibiotic); Antibacterial protein LL-37] |
| P80511 | | S100 protein | Protein S100-A12 (CGRP) (Calcium-binding protein in amniotic fluid 1) (CAAF1) (Calgranulin-C) (CAGC) (Extracellular newly identified RAGE-binding protein) (EN-RAGE) (Neutrophil S100 protein) (S100 calcium-binding protein A12) (p6) [Cleaved into: Calcitermin] |
| P05109 | P05109 | S100 protein | Protein S100-A8 (Calgranulin-A) (Calprotectin L1L subunit) (Cystic fibrosis antigen) (CFAG) (Leukocyte L1 complex light chain) (Migration inhibitory factor-related protein 8) (MRP-8) (p8) (S100 calcium-binding protein A8) (Urinary stone protein band A) |
| P06702 | P06702 | S100 protein | Protein S100-A9 (Calgranulin-B) (Calprotectin L1H subunit) (Leukocyte L1 complex heavy chain) (Migration inhibitory factor-related protein 14) (MRP-14) (p14) (S100 calcium-binding protein A9) |
| | P31151# | S100 protein | S100A7. Psoriasin, S100 calcium-binding protein A7 |
| P02775 | | Cytotoxin | Platelet basic protein (PBP) (C-X-C motif chemokine 7) (Leukocyte-derived growth factor) (LDGF) (Macrophage-derived growth factor) (MDGF) (Small-inducible cytokine B7) [Cleaved into: Connective tissue-activating peptide III (CTAP-III) (LA-PF4) (Low-affinity platelet factor IV); TC-2; Connective tissue-activating peptide III(1-81) (CTAP-III(1-81)); Beta-thromboglobulin (Beta-TG); Neutrophil-activating peptide 2(74) (NAP-2(74)); Neutrophil-activating peptide 2(73) (NAP-2(73)); Neutrophil-activating peptide 2 (NAP-2); TC-1; Neutrophil-activating peptide 2(1-66) (NAP-2(1-66)); Neutrophil-activating peptide 2(1-63) (NAP-2(1-63))] |
| P78556 | | Cytotoxin | C-C motif chemokine 20 (Beta-chemokine exodus-1) (CC chemokine LARC) (Liver and activation-regulated chemokine) (Macrophage inflammatory protein 3 alpha) (MIP-3-alpha) (Small-inducible cytokine A20) [Cleaved into: CCL20(1-67); CCL20(1-64); CCL20(2-70)] |
| P13727 | | Cytotoxin | Bone marrow proteoglycan (BMPG) (Proteoglycan 2) [Cleaved into: Eosinophil granule major basic protein (EMBP) (MBP) (Pregnancy-associated major basic protein)] |
| Q9NZH7 | | Cytotoxin | Interleukin-36 beta (FIL1 eta) (Interleukin-1 eta) (IL-1 eta) (Interleukin-1 family member 8) (IL-1F8) (Interleukin-1 homolog 2) (IL-1H2) |
| P20160 | | Serine Protease, Peptidase S1 | Azurocidin (Cationic antimicrobial protein CAP37) (Heparin-binding protein) (HBP) |
| P08311 | P08311 | Serine Protease, Peptidase S1 | Cathepsin G (CG) (EC 3.4.21.20) |
| Q9NRD9 | | Dual oxidase | Dual oxidase 1 (EC 1.11.1.-) (EC 1.6.3.1) (Large NOX 1) (Long NOX 1) (NADPH thyroid oxidase 1) (Thyroid oxidase 1) |
| Q9NRD8 | | Dual oxidase | Dual oxidase 2 (EC 1.11.1.-) (EC 1.6.3.1) (Large NOX 2) (Long NOX 2) (NADH/NADPH thyroid oxidase p138-tox) (NADPH oxidase/peroxidase DUOX2) (NADPH thyroid oxidase 2) (Thyroid oxidase 2) (p138 thyroid oxidase) |
| P20749 | | transcription regulation | B-cell lymphoma 3 protein (BCL-3) (Proto-oncogene BCL3) |
| 075594 | | TBD | Peptidoglycan recognition protein 1 (Peptidoglycan recognition protein short) (PGRP-S) |
| Q96LB9 | | TBD | Peptidoglycan recognition protein 3 (Peptidoglycan recognition protein I-alpha) (PGLYRPIalpha) (PGRP-I-alpha) (Peptidoglycan recognition protein intermediate alpha) |
| Q96LB8 | | TBD | Peptidoglycan recognition protein 4 (Peptidoglycan recognition protein I-beta) (PGLYRPIbeta) (PGRP-I-beta) (Peptidoglycan recognition protein intermediate beta) |
| Q9H112 | | TBD | Cystatin-11 |
| P12724 | | TBD | Eosinophil cationic protein (ECP) (EC 3.1.27.-) (Ribonuclease 3) (RNase 3) |
| P22749 | | TBD | Granulysin (Lymphokine LAG-2) (Protein NKG5) (T-cell activation protein 519) |
| P81172 | | TBD | Hepcidin (Liver-expressed antimicrobial peptide 1) (LEAP-1) (Putative liver tumor regressor) (PLTR) [Cleaved into: Hepcidin-25 (Hepc25); Hepcidin-20 (Hepc20)] |
| P02788 | | TBD | Lactotransferrin (Lactoferrin) (EC 3.4.21.-) (Talalactoferrin) [Cleaved into: Kaliocin-1; Lactoferroxin-A; Lactoferroxin-B; Lactoferroxin-C] |
| P18428 | | TBD | Lipopolysaccharide-binding protein (LBP) |
| Q969E1 | | TBD | Liver-expressed antimicrobial peptide 2 (LEAP-2) |
| P61626 | | TBD | Lysozyme C (EC 3.2.1.17) (1,4-beta-N-acetylmuramidase C) |
| Q96FL8 | | TBD | Multidrug and toxin extrusion protein 1 (MATE-1) (hMATE-1) (Solute carrier family 47 member 1) |
| Q86VL8 | | TBD | Multidrug and toxin extrusion protein 2 (MATE-2) (hMATE-2) (Kidney-specific H(+)/organic cation antiporter) (Solute carrier family 47 member 2) |
| P10153 | | TBD | Non-secretory ribonuclease (EC 3.1.27.5) (Eosinophil-derived neurotoxin) (RNase Upl-2) (Ribonuclease 2) (RNase 2) (Ribonuclease US) |
| Q9H1E1 | Q9H1E1 | TBD | Ribonuclease 7 (RNase 7) (EC 3.1.27.-) (Skin-derived antimicrobial protein 2) (SAP-2) |
| P55085 | | TBD | Proteinase-activated receptor 2 (PAR-2) (Coagulation factor II receptor-like 1) (G-protein coupled receptor 11) (Thrombin receptor-like 1) [Cleaved into: Proteinase-activated receptor 2, alternate cleaved 1; Proteinase-activated receptor 2, alternate cleaved 2] |
| P60602 | | TBD | Reactive oxygen species modulator 1 (ROS modulator 1) (Epididymis tissue protein Li 175) (Glyrichin) (Mitochondrial targeting GxxxG motif protein) (MTGM) (Protein MGR2 homolog) |
| Q9NQ38 | | TBD | Serine protease inhibitor Kazal-type 5 (Lympho-epithelial Kazal-type-related inhibitor) (LEKTI) [Cleaved into: Hemofiltrate peptide HF6478; Hemofiltrate peptide HF7665] |
| P02808 | | TBD | Statherin |
| Q8WWY7 | | TBD | WAP four-disulfide core domain protein 12 (Putative protease inhibitor WAP12) (Whey acidic protein 2) |

| | | | |
|---|---|---|---|
| # Proteins not yet included in UniProt AMP list, but their antimicrobial function has been referenced (ref) | | | |

**Table 2**

| **Entry** | **Protein names** | **D:ND** | **p-value** | **ZPT:D** | **p-value** | **ZPT:ND** | **p-value** | **Selenium Sulfide:D** | **p-value** | **Selenium Sulfide:ND** | **p-value** |
|---|---|---|---|---|---|---|---|---|---|---|---|
| Q99879 | Histone H2B type 1-M | 1.870 | 0.000 | 0.420 | 0.000 | 0.785 | 0.152 | 0.621 | 0.005 | 1.160 | 0.337 |
| P16104 | Histone H2A | 1.441 | 0.154 | 0.584 | 0.025 | 0.842 | 0.505 | 0.718 | 0.263 | 1.035 | 0.913 |
| P62805 | Histone H4 | 1.190 | 0.200 | 0.720 | 0.090 | 1.010 | 0.950 | 0.710 | 0.061 | 0.990 | 0.950 |
| P31151 | Protein S100-A7 | 1.502 | <0.0001 | 0.705 | <0.0001 | 1.059 | 0.433 | 0.844 | 0.021 | 1.268 | 0.001 |
| P05109 | Protein S100-A8 | 1.612 | <0.0001 | 0.838 | 0.001 | 1.351 | <0.0001 | 0.763 | <0.0001 | 1.231 | <0.0001 |
| P06702 | Protein S100-A9 | 1.476 | <0.0001 | 0.935 | 0.181 | 1.381 | <0.0001 | 0.811 | <0.0001 | 1.197 | <0.0001 |
| P08311 | Cathepsin G | 1.503 | 0.083 | 0.934 | 0.800 | 1.404 | 0.190 | 0.696 | 0.108 | 1.050 | 0.810 |
| P59666 | Neutrophil defensin 3 (Defensin, alpha 3) (HNP-3) | 1.587 | 0.005 | 0.372 | <0.0001 | 0.590 | 0.002 | 0.759 | 0.107 | 1.205 | 0.202 |
| P81605 | Dermcidin | 0.266 | 0.000 | 2.112 | <0.0001 | 0.562 | <0.0001 | 2.142 | <0.0001 | 0.570 | <0.0001 |
| Q9H1E1 | Ribonuclease 7 (RNase 7) | 1.575 | 0.040 | 0.736 | 0.100 | 1.159 | 0.487 | 0.746 | 0.071 | 1.175 | 0.398 |
| P81534 | Beta-defensin 103 (hBD-3) | 1.239 | 0.0554 | 0.878 | 0.1365 | 1.045 | 0.6107 | NA | NA | NA | NA |

| | | | | | | | | | | | |
|---|---|---|---|---|---|---|---|---|---|---|---|
| **Key:** **D=dandruff** **ND= Non-Dandruff** **ZPT=1% ZPT shampoo** **Selenium sulfide = 1% selenium sulfide shampoo** **NA=not analyzed** | | | | | | | | | | | |

## Claims

1. A noninvasive method for diagnosing dandruff in a subject comprising:
a) collecting an epithelial cell /skin cell sample from the subject;
b) detecting a level of an antimicrobial peptide biomarker being human beta defensin-2 (hBD2) biomarker in the epithelial cell sample/skin cell sample;
c) diagnosing the subject as having dandruff based on the level of human-beta defensin-2, by determining the amount of the human beta defensin-2 (hBD2) biomarker in the epithelial cells as compared to a normal population.

2. The noninvasive method for diagnosing dandruff in a subject according to claim 1 comprising:
a) applying an adhesive article to an epithelium of a mammal;
b) allowing for adherence of epithelial cells to the adhesive article;
c) removing the adhesive article from the epithelium of the mammal;
d) preparing the adhesive article using standard laboratory methods for extraction;
e) extracting a human beta defensin-2 (hBD2) biomarker from the epithelial cells adhered to said adhesive article;
f) measuring the human beta defensin-2 (hBD2) biomarker from the epithelial cells adhered to said adhesive article;
g) determining the amount of the human beta defensin-2 (hBD2) biomarker in the epithelial cells following a treatment as compared to a baseline sample.

3. The method according any preceding claims wherein collection of skin sample is from the group consisting of adhesive articles, hair plucks, skin wash and mixtures thereof.

4. The method according any preceding claims, wherein the level of the antimicrobial peptide biomarker is standardized by dividing the biomarker by an amount of protein on the adhesive article.

5. The method of according any preceding claims, wherein the epithelium comprises stratum corneum.

6. The method of according any preceding claims, wherein there is a change in biomarker level following treatment when compared to a baseline level of biomarker.

7. The method of according any preceding claims, wherein there is a change from baseline in standardized biomarker following application with an antifungal hair treatment, when compared to a baseline level of biomarker prior to the application.

8. The method of according any preceding claims, wherein there is a 33% reduction in standardized hBD2 biomarker following treatment when compared to a baseline level of hBD2 biomarker prior to the application.

9. The method of according any preceding claims, wherein there is a change in standardized biomarker following application with a zinc pyrithione shampoo when compared to a baseline level of biomarker prior to the application.

10. The method of according any preceding claims, wherein there is a change in standardized molecule biomarker following application with a selenium sulfide shampoo when compared to a baseline level of biomarker prior to the application.

11. The method of according any preceding claims, wherein there is improvement of at least 5% in dandruff compared to a normal population.

12. The method of according any preceding claims, wherein there is at least a 5% difference between a dandruff treatment and a non-dandruff treatment in a dandruff population.

13. The method of according any preceding claims, wherein the mammal is a human.

## Patentansprüche

1. Nicht-invasives Verfahren zum Diagnostizieren von Schuppen bei einem Probanden, umfassend:
a) Sammeln einer Epithelzell-/Hautzellprobe von dem Probanden;
b) Detektieren eines Niveaus eines antimikrobiellen Peptid-Biomarkers, der ein menschlicher Beta-Defensin-2 (hBD2)-Biomarker ist, in der Epithelzell-/Hautzellprobe;
c) Diagnostizieren des Probanden mit Schuppen auf dem Niveau von Human-Beta-Defensin-2, durch Bestimmen der Menge des Biomarkers Human-Beta-Defensin-2 (hBD2) in den Epithelzellen im Vergleich zu einer normalen Population.

2. Nicht-invasive Verfahren zum Diagnostizieren von Schuppen bei einem Probanden nach Anspruch 1, umfassend:
a) Auftragen eines Klebstoffartikels auf ein Epithel eines Säugetiers;
b) Ermöglichen des Anhaftens von Epithelzellen an dem Klebstoffartikel;
c) Entfernen des Klebstoffartikels von dem Epithel des Säugetiers;
d) Vorbereiten des Klebstoffartikels mittels Standard-Laborverfahren zur Extraktion;
e) Extrahieren eines menschlichen Beta-Defensin-2 (hBD2)-Biomarkers aus den Epithelzellen, die an dem Klebstoffartikel haften;
f) Messen des menschlichen Beta-Defensin-2 (hBD2)-Biomarkers aus den Epithelzellen, die an dem Klebstoffartikel haften;
g) Bestimmen der Menge des menschlichen Beta-Defensin-2 (hBD2)-Biomarkers in den Epithelzellen nach einer Behandlung im Vergleich zu einer Ausgangsprobe.

3. Verfahren nach einem der vorstehenden Ansprüche, wobei das Nehmen der Hautprobe aus der Gruppe, bestehend aus Klebeartikeln, Haarausrissen, Hautwäsche und Mischungen davon, stammt.

4. Verfahren nach einem der vorstehenden Ansprüche, wobei das Niveau des antimikrobiellen Peptid-Biomarkers durch Dividieren des Biomarkers durch eine Proteinmenge an dem Klebeartikel standardisiert wird.

5. Verfahren nach einem der vorstehenden Ansprüche, wobei das Epithel Stratum corneum umfasst.

6. Verfahren nach einem der vorstehenden Ansprüche, wobei eine Änderung des Biomarker-Niveaus nach der Behandlung im Vergleich zu einem Ausgangsniveau des Biomarkers vorliegt.

7. Verfahren nach einem der vorstehenden Ansprüche, wobei eine Änderung vom Ausgangswert des standardisierten Biomarkers nach Anwendung einer pilzbefallverhütenden Haarbehandlung im Vergleich zu einem Ausgangsniveau des Biomarkers vor der Anwendung vorliegt.

8. Verfahren nach einem der vorstehenden Ansprüche, wobei eine 33 %-ige Reduzierung des standardisierten hBD2-Biomarkers nach der Behandlung im Vergleich zu einem Ausgangsniveau des hBD2-Biomarkers vor der Anwendung vorliegt.

9. Verfahren nach einem der vorstehenden Ansprüche, wobei eine Änderung des standardisierten Biomarkers nach Anwendung eines Zink-Pyrithion-Shampoos im Vergleich zu einem Ausgangsniveau des Biomarkers vor der Anwendung vorliegt.

10. Verfahren nach einem der vorstehenden Ansprüche, wobei eine Änderung des standardisierten Molekül-Biomarkers nach Anwendung eines Selensulfid-Shampoos im Vergleich zu einem Ausgangsniveau des Biomarkers vor der Anwendung vorliegt.

11. Verfahren nach einem der vorstehenden Ansprüche, wobei eine Verbesserung der Schuppenbildung von mindestens 5 % im Vergleich zu einer normalen Population vorliegt.

12. Verfahren nach einem der vorstehenden Ansprüche, wobei eine Differenz von mindestens 5 % zwischen einer Schuppenbehandlung und einer Nicht-Schuppenbehandlung in einer Schuppenpopulation vorliegt.

13. Verfahren nach einem der vorstehenden Ansprüche, wobei das Säugetier ein Mensch ist.

## Revendications

1. Procédé non invasif de diagnostic de pellicules chez un sujet comprenant :
a) la collecte d'un échantillon de cellule épithéliale/cellule de peau chez le sujet ;
b) la détection d'un taux d'un biomarqueur peptidique antimicrobien étant un biomarqueur de bêta défensine-2 humaine (hBD2) dans l'échantillon de cellule épithéliale/échantillon de cellule de peau ;
c) le diagnostic du sujet comme ayant des pellicules sur la base du niveau de bêta défensine-2 humaine, en déterminant la quantité du biomarqueur de bêta défensine-2 humaine (hBD2) dans les cellules épithéliales par rapport à une population ordinaire.

2. Procédé non invasif de diagnostic de pellicules chez un sujet selon la revendication 1 comprenant :
a) appliquer un article adhésif sur un épithélium d'un mammifère ;
b) permettre l'adhérence des cellules épithéliales à l'article adhésif ;
c) retirer l'article adhésif de l'épithélium du mammifère ;
d) préparer l'article adhésif en utilisant des procédés standard de laboratoire pour l'extraction ;
e) extraire un biomarqueur de bêta défensine-2 humaine (hBD2) des cellules épithéliales mises en adhésion audit article adhésif ;
f) mesurer le biomarqueur de bêta défensine-2 humaine (hBD2) des cellules épithéliales mises en adhésion audit article adhésif ;
g) déterminer la quantité du biomarqueur de bêta défensine-2 humaine (hBD2) dans les cellules épithéliales à la suite d'un traitement par rapport à un échantillon de référence.

3. Procédé selon l'une quelconque des revendications précédentes, dans lequel la collecte d'échantillon de peau provient du groupe constitué d'articles adhésifs, d'arrachages de cheveux, de lavage de peau et de leurs mélanges.

4. Procédé selon l'une quelconque des revendications précédentes, dans lequel le taux du biomarqueur peptidique antimicrobien est normalisé en divisant le biomarqueur par une quantité de protéine sur l'article adhésif.

5. Procédé selon l'une quelconque des revendications précédentes, dans lequel l'épithélium comprend la couche cornée.

6. Procédé selon l'une quelconque des revendications précédentes, dans lequel il y a un changement de taux de biomarqueur à la suite d'un traitement par comparaison avec un taux de référence de biomarqueur.

7. Procédé selon l'une quelconque des revendications précédentes, dans lequel il y a un changement par rapport aux valeurs de référence dans le biomarqueur normalisé à la suite d'une application avec un traitement capillaire antifongique, par comparaison avec un taux de référence de biomarqueur avant l'application.

8. Procédé selon l'une quelconque des revendications précédentes, dans lequel il y a une réduction de 33 % du biomarqueur hBD2 normalisé à la suite d'un traitement par comparaison avec un taux de référence de biomarqueur hBD2 avant l'application.

9. Procédé selon l'une quelconque des revendications précédentes, dans lequel il y a un changement dans le biomarqueur normalisé à la suite d'une application avec un shampooing à la pyrithione de zinc par comparaison avec un taux de référence de biomarqueur avant l'application.

10. Procédé selon l'une quelconque des revendications précédentes, dans lequel il y a un changement dans le biomarqueur de molécule normalisé à la suite d'une application avec un shampoing au sulfure de sélénium par comparaison avec un taux de référence de biomarqueur avant l'application.

11. Procédé selon l'une quelconque des revendications précédentes, dans lequel il y a une amélioration d'au moins 5 % des pellicules par comparaison avec une population ordinaire.

12. Procédé selon l'une quelconque des revendications précédentes, dans lequel il y a au moins une différence de 5 % entre un traitement des pellicules et un non-traitement des pellicules dans une population présentant des pellicules.

13. Procédé selon l'une quelconque des revendications précédentes, dans lequel le mammifère est un humain.
